# FASCICULE DE BREVET EUROPEEN

(11) **EP 2 331 020 B1**
(45) Date de publication et mention de la délivrance du brevet: **04.07.2012**
(21) Numéro de dépôt: 09740175.6
(22) Date de dépôt: 16.09.2009
(51) Int. Cl.: A61F 2/36

(54) **TIGE FEMORALE DE PROTHESE DE HANCHE**
FEMURSTANGE FÜR EINE HÜFTPROTHESE
FEMORAL ROD FOR A HIP PROSTHESIS

(30) Priorité: 17.09.2008 FR 0805103; 27.10.2008 FR 0805963
(43) Date de publication de la demande: 15.06.2011
(73) Titulaire: T.O., 69002 Lyon (FR)
(72) Inventeur: THEILLIEZ, Boris, F-26300 Bourg de Péage (FR); BAULOT, Emmanuel, F-21000 Dijon (FR); FARISON, Frederic, F-42400 Saint Chamond (FR); DOURSOUNIAN, Levon, F-75016 Paris (FR); PHILIPPOT, Rémi, F-42000 Saint Etienne (FR); FAVRE, Eric, F-73000 Chambéry (FR); LIMOZIN, Rodolphe, F-12850 Onet Le Chateau (FR); PROVOST, Gilles, F-01240 Certines (FR); DOYCINOVICH, Serge, F-01000 Bourg en Bresse (FR); VANEL, Olivier, F-43220 Dunières (FR); COSTES, Cédric, F-87000 Limoges (FR); CAMILLERI, Jean Philippe, F-69008 Lyon (FR); FERON, Jean Marc, F-94300 Vincennes (FR); CHANZY, Nicolas, F-92100 Boulogne Billancourt (FR)
(74) Mandataire: Schmidt, Martin Peter
(86) Numéro de dépôt international: PCT/FR2009/001099
(87) Numéro de publication internationale: WO 2010/031922

(56) Documents cités:
- EP-A- 0 078 888
- EP-A- 0 159 510
- EP-A- 1 639 964
- WO-A-2004/064676
- WO-A-2005/034817
- DE-A1- 2 627 569
- DE-A1- 19 811 820
- FR-A- 2 600 527
- FR-A- 2 641 462
- FR-A- 2 892 922

## Description

### Domaine de l'invention

La présente invention concerne les prothèses de hanche, plus particulièrement les tiges fémorales et les pivots fémoraux pour tiges fémorales de prothèses de hanche.

### Etat de la technique

Une tige fémorale présente généralement une partie médullaire de forme générale rectiligne ou légèrement incurvée, appelée pivot fémoral, destinée à être engagée dans la cavité médullaire du fémur, un col incliné surmontant cette partie médullaire, et une tête sphérique située dans le prolongement du col. La tête sphérique peut être réalisée en une pièce monobloc avec la tige ou elle peut être engagée par emmanchement sur l'extrémité conique du col.

Lorsque la tige fémorale est mise en place, le col devient protubérant à l'extérieur de l'os et la tête sphérique est alors engagée soit dans la cavité cotyloïdienne de la hanche (éventuellement couplée à une cupule intermédiaire), dans le cas d'une prothèse fémorale, soit dans un cotyle fixé dans le bassin, dans le cas d'une prothèse totale de hanche.

La mise en place de la tige dans le fémur s'effectue généralement par deux techniques : une première technique selon laquelle la tige est cimentée par adjonction d'un ciment orthopédique qui colle le pivot fémoral au fémur; et une deuxième technique dans laquelle la tige n'est pas cimentée, mais elle est fixée en force dans l'os. Les surfaces du pivot fémoral peuvent comporter un revêtement de surface, par exemple du hydroxyapatite de calcium (HAC) ou titane poreux qui permet à l'os de coloniser le pivot fémoral, ou elles peuvent subir un traitement de surface.

La mise en place de la tige fémorale dans la cavité fémorale doit répondre aux exigences d'assise efficace de la tige fémorale dans la cavité médullaire, apte à supporter des contraintes de torsion, de rotation et d'enfoncement.

Pour remédier à ce problème, une solution a été de proposer des tiges fémorales présentant des faces antérieure et postérieure inclinées par rapport au plan de symétrie de la prothèse, leurs surfaces étant plus importantes dans la partie proximale que dans la partie distale de la tige. Si cette forme améliore l'appui de la tige fémorale sur l'os, sa mise en place nécessite de consommer beaucoup de capital osseux. Par conséquent, un risque d'éclatement de l'os fémoral est toujours présent avec une telle tige fémorale. De surcroît, une éventuelle reprise s'avère très difficile par la suite.

Le document US 7 175 668 décrit une tige fémorale présentant différentes formes de section transversale d'ancrage, chaque section présentant quatre côtés principaux d'appui, des évidements pouvant être réalisés sur certains d'entre eux. Elle présente, certes, une surface de contact avec la cavité osseuse augmentée, sa mise en place consomme beaucoup de capital osseux. Les sections d'ancrage proposées ont différentes formes, mais présentent un même inconvénient qui est leur faible résistance aux sollicitations mécaniques comme conséquence d'une une section très affaiblie, qu'elle soit creuse ou pleine. Par ailleurs, les coins à angles vifs de ces sections constituent des concentrateurs d'efforts qui, transmis ensuite à l'os, sont susceptibles de provoquer des fissures pouvant mener à éclatement de celui-ci. De surcroît, de telles sections à angle vif nécessitent des opérations de finition qui augmentent le coût final de la prothèse.

Une autre solution est connue du document FR 2 675 040 où la tige fémorale présente une section transversale d'ancrage de forme générale elliptique. Une telle section exempte de coin tranchant pose, certes, moins de problème quant à la concentration de l'effort sur la tranche, et consomme moins de capital osseux, mais elle présente toutefois l'inconvénient d'une assise de faible surface, mais surtout d'une faible résistance aux couples de rotation. Par ailleurs, une quantité importante de ciment est nécessaire pour sa fixation, ciment qui n'arrive pas toujours être bien réparti entre la tige et les parois de la cavité médullaire.

Le document WO 2004/064676 décrit une tige fémorale dont une ou plusieurs faces de sa partie distale comprennent au moins une cannelure, une fente sagittale ou une rainure en hélice. Une telle tige fémorale présente, certes, une meilleure résistance aux sollicitations en torsion de la tige, mais au détriment de la surface de contact avec l'os qui se trouve, elle, diminuée.

### Objet de l'invention

Le but de l'invention est de remédier à ces inconvénients et de proposer une tige fémorale pour prothèse de hanche qui permet une fixation solide et durable dans l'os, sans possibilité de rotation de la tige à l'usage, et qui offre en même temps une surface de contact suffisante avec les parois osseuses de la cavité médullaire.

Un autre but de l'invention est de proposer une tige fémorale qui, en cas d'éventuelle reprise fémorale permet une ablation de l'implant sans nuire au capital osseux.

Un autre but de l'invention est de proposer une tige fémorale qui améliore la repousse durable des cellules osseuses sur le pourtour de la prothèse convenablement traitée en surface.

Un autre but de l'invention est une tige fémorale qui soit efficace et fiable en fonctionnement, tout en pouvant être fabriquée de manière économique, typiquement par des techniques de forgeage ou de moulage.

Ces buts sont atteints avec un pivot fémoral pour une tige fémorale de prothèse de hanche, ledit pivot comportant une partie métaphysaire proximale prolongée vers la bas par une partie diaphysaire distale, chacune des parties métaphysaire et diaphysaire présentant une section transversale d'ancrage dans le fémur ayant un contour extérieur de forme générale rectangulaire, section qui va en se rétrécissant de la partie proximale vers la partie distale et ledit pivot étant caractérisé en ce que la section transversale d'ancrage présente un contour rogné et que les côtés du contour sont recourbés vers son centre suivant un arc d'ellipse sur la majeure partie de la partie diaphysaire.

Par pivot fémoral on comprend la partie d'une tige fémorale ne comportant pas de col. Un tel pivot fémoral peut être fixé par ciment ou en force dans la cavité médullaire préalablement préparée par un chirurgien.

Selon l'invention, la section transversale d'ancrage du pivot fémoral présente un contour extérieur de forme générale rectangulaire rogné, en ayant ses côtés recourbés vers le centre du contour, et ceci sur la majeure partie de la longueur de la partie diaphysaire. Par section transversale d'ancrage, on comprend une section perpendiculaire à l'axe longitudinal du pivot fémoral. Par contour rogné de cette section, on comprend un contour de forme générale rectangulaire dont les côtés on été diminués d'une petite partie, suivant une forme en arc, en les faisant avancer vers l'intérieur de la section. Cette section est alors un rectangle dont le contour décrit des arcs reliant les coins du rectangle. Lorsque la tige présente des stries ou rainures sur les faces de la partie diaphysaire, par contour de sa section transversale on comprend la courbe enveloppe de ses crêtes. Ainsi, un tel contour de forme générale rectangulaire (qui pourrait également être un carré ou un losange), permet déjà d'offrir une bonne résistance aux sollicitations de rotation et de torsion de la tige fémorale, ces sollicitations étant notamment supportées au niveau des coins du rectangle. De surcroît, un tel contour extérieur rogné permet d'augmenter la surface de contact entre l'os et l'implant, tout en gardant du capital osseux. En effet, pour une surface de contact équivalente, en creusant la surface d'ancrage de la tige, on enlève moins de tissu osseux, ce qui permet d'avoir une paroi osseuse plus importante et, donc, plus résistante, ainsi que de la réserve osseuse pour une éventuelle reprise de prothèse.

Une telle section d'ancrage de la partie diaphysaire de l'implant selon l'invention permet alors de ménager les parois de faible épaisseur de la cavité médullaire du fémur, tout en présentant une bonne assise et une bonne résistance aux sollicitations mécaniques et ceci sur la majeure partie de la longueur de la partie d'ancrage de l'implant. Par majeure partie de la partie diaphysaire, on comprend une longueur de celle-ci s'étendant à partir de la zone de jonction entre la partie métaphysaire et la partie diaphysaire de l'implant et allant jusqu'au-dessus de l'extrémité distale libre de la partie diaphysaire sur au moins deux de ses faces latérales en vis-à-vis, ou se continuant jusqu'à l'extrémité distale libre de la partie diaphysaire pour les deux autres faces latérales en vis-à-vis.

Selon l'invention également, les côtés du contour de la section transversale sont recourbés vers le centre suivant un arc d'ellipse. Chaque côté du contour est ainsi recourbé vers le centre de la section suivant un arc d'ellipse, car on arrive ainsi à augmenter la surface de contact entre l'os et la prothèse, tout en permettant d'obtenir un pivot fémoral plus résistant aux sollicitations mécaniques, notamment aux efforts de compression. Par ailleurs, il a été constaté que, pour les tiges implantées avec du ciment, une telle forme de la section transversale du pivot arrivait à mieux mettre en compression le ciment lorsqu'elle est mise en place à l'intérieur de la cavité médullaire.

Avantageusement, la surface de la face antérieure et celle de la face postérieure de la partie diaphysaire sont obtenues chacune par l'intersection d'un plan incliné faisant un angle α avec le plan de symétrie du pivot avec un tronc de cône générateur d'axe incliné faisant un angle β avec le plan de symétrie du pivot, la génératrice du cône étant tangente au plan de la face antérieure, respectivement de la face postérieure sur toute sa longueur.

Le contour extérieur de forme générale rectangulaire de la section transversale d'ancrage dans le fémur, est défini par une coupe transversale du pivot fémoral qui présente, de manière connue quatre faces : une face avant ou antérieure, une face arrière opposée à la première ou face postérieure et une face interne, orientée vers l'intérieur du corps et une face externe, faisant face à la face interne.
On aurait pu, certes, utiliser un cylindre générateur pour obtenir les côtés recourbés des faces antérieure et postérieure. Le profil de section à côtés recourbés obtenu avec un tronc de cône générateur présente avantageusement un rayon de raccordement plus important à l'extrémité inférieure de la partie diaphysaire qu'à sa partie supérieure. Ceci permet d'obtenir de manière aisée des profils dont les côtés sont de plus en plus recourbés vers le haut et d'augmenter ainsi progressivement la surface de contact avec l'os. Un pivot fémoral ainsi obtenu présente une section plus robuste sur toute la longueur de sa partie diaphysaire, ce qui lui confère une meilleure résistance aux sollicitations mécaniques, tout en pouvant être extrait facilement de l'os en cas de besoin.

De préférence, pour une section transversale prédéfinie, les côtés des faces antérieure et postérieure présentent un même rayon de courbure qui est supérieur à celui des faces intérieure et extérieure.

Les faces interne et externe ont généralement des dimensions moindres (par exemple leur largeur) que celles antérieure et postérieure et, de ce fait, un ajustement de leur côté avec un rayon moins important permet d'augmenter la surface de contact, même pour ces côtés de faible largeur. A titre d'exemple, pour une section transversale donnée, le rapport entre le rayon de courbure du cercle générateur des faces antérieure et postérieure et celui des faces interne et externe est avantageusement compris entre 1,2 et 2, encore plus avantageusement entre 1,3 et 1,7 et de préférence égal à 1,4.

De préférence, les sections transversales successives de la section d'ancrage présentent des contours arrondis au niveau des faces antérieure et postérieure sur toute la longueur de la partie diaphysaire, alors que le contour des faces interne et externe est arrondi sur la majeure partie de la longueur de la partie diaphysaire, sauf à quelques millimètres (pouvant aller jusqu'à ou quelques centimètres) au dessus de l'extrémité distale de la partie diaphysaire ou il présente des lignes droites.
Cette forme préférée de réalisation permet d'obtenir une partie d'extrémité distale dont les côtés interne et externe sont renforcés par des droites, assurant ainsi une meilleure fixation du pivot dans l'os, notamment spongieux, à son extrémité distale. Le pivot fémoral de l'invention est fixé dans l'os de manière à recevoir les contraintes principalement dans la zone de la partie métaphysaire proximale et dans celle de l'extrémité distale de la partie diaphysaire. La partie diaphysiare à contour rogné reliant ces deux extrémités de fixation sert alors de répartiteur de contraintes appliquées à l'os.

Avantageusement, le contour extérieur de la section transversale d'ancrage présente des coins arrondis.

Une telle forme galbée de la section transversale du pivot fémoral permet d'éviter toute partie anguleuse pouvant être le siège d'efforts de rupture ou d'endommagement de l'os lors de la mise en place de l'implant, tout en lui permettant de garder de bonnes propriétés de résistance en rotation et torsion. Un tel rayon de raccordement peut être compris entre 2 et 5mm, et il est de préférence égal à environ 3,5 mm.

De préférence, la partie métaphysaire présente une forme évasée en direction d'une cavité de col.

Une telle forme de la partie métaphysaire assure une bonne compression du ciment en évitant la formation de bulles d'air entre les partie cimentées, lorsque l'implant est mis en place par adjonction de ciment, ainsi qu'une facilité de l'insertion du matériel de reprise (par exemple des ciseaux, des mèches, etc.) entre l'os et la prothèse, en cas de reprise de celle-ci.

Avantageusement, la partie métaphysaire présente des nervures horizontales et des rainures horizontales sensiblement sur la totalité de la surface de ses faces interne, antérieure et postérieure.

Ces nervures et rainures horizontales (ou agencées perpendiculairement à l'axe longitudinal du pivot fémoral) assurent une bonne résistance aux sollicitations d'enfoncement et de rotation de la prothèse, elles augmentent la surface de contact entre celle-ci et l'os et, par ailleurs, elle favorisent la repousse osseuse sur le pourtour de la tige.

De préférence, la partie diaphysaire comprend des rainures verticales sur ses quatre faces.

Ces rainures verticales, parallèles à l'axe longitudinal de la tige, assurent un bon guidage de la prothèse lors de sa mise en place, tout en augmentant la surface entre l'os et l'implant. Dans une version de mise en place sans ciment, ces rainures verticales permettent un emboîtement autobloquant de l'implant dans l'os.

Avantageusement, le pivot fémoral selon l'invention comporte une cavité de réception d'un col amovible.

Par col amovible, on comprend un col séparable du pivot fémoral dont la base est prévue pour être emmanchée dans la cavité du pivot, le sommet étant, lui, destiné à recevoir une tête sphérique. Un tel col amovible permet d'adapter, pour un pivot fémoral donné, un col ayant des dimensions et un angle d'inclinaison proches de la géométrie anatomique du patient. On obtient ainsi une palette plus large d'implants avec une possibilité d'adaptation plus fine de l'implant aux divers patients. Par ailleurs, un col monté amovible dans un pivot fémoral permet de remplacer uniquement le col (et non pas la tige complète) au cas où celui-ci viendrait à être endommagé, avec une intervention chirurgicale simplifiée et sans ablation de l'os.

De préférence, la partie métaphysaire comprend un orifice taraudé de réception d'organe extracteur ou impacteur.

Ceci offre des facilités pour positionner rapidement un organe extracteur en cas de reprise de prothèse, mais également pour positionner un organe impacteur fémoral en cas de mise en place de la prothèse. Cet orifice est avantageusement situé sur la surface frontale supérieure de la partie métaphysaire.

Avantageusement, avec le pivot fémoral de l'invention, on peut obtenir une gamme de pivots fémoraux, comprenant une pluralité de pivots fémoraux dont les dimensions au moins de leur longueur et des paramètres définissant le profil de la section transversale pour les faces antérieure, postérieure et externe de chaque pivot sont établies en progression homothétique.

Une telle gamme peut par exemple comprendre douze pivots, leurs longueur pouvant aller, par exemple de 120 mm à 190 mm, les dimensions des paramètres définissant le profil de la section transversale pour les faces antérieure, postérieure et externe variant de manière homothétique de 0.7mm ou de 1mm d'un pivot à l'autre. Ceci permet une adaptation fidèle aux différents morphotypes des patients. Avec une telle gamme de pivots fémoraux à laquelle se rajoute une gamme de cols amovibles on peut multiplier le nombre de prothèses et obtenir ainsi une gamme de prothèses couvrant un grand nombre de morphotypes.

L'objet de l'invention est également réalisé avec une tige fémorale comportant un pivot fémoral de l'invention complété par un col et une tête sphérique.

L'invention est applicable aussi bien aux prothèses fémorales qu'au prothèses totales de hanche.

### Description des figures

Les figures 1 à 2 se réfèrent à un premier mode de réalisation de l'invention, où :
- la figure 1a est une vue de côté du pivot fémoral de l'invention et la figure 1 b est une vue en coupe selon le plan A-A de la figure 1a ;
- la figure 2a illustre une vue en perspective, la figure 2b une vue frontale de la face externe et la figure 2c une vue de dessous du pivot fémoral de l'invention et des surfaces génératrices des ses côtés ;

Les figures 3 à 7 se réfèrent à un deuxième mode de réalisation de l'invention, où :
- la figure 3a illustre une vue frontale de la face externe et la figure 3b une vue de côté du pivot fémoral selon l'invention ;
- la figure 4a est une vue frontale de la face interne du pivot fémoral de l'invention et la figure 4b une vue à échelle agrandie du détail h de la figure 4a ;
- la figure 5a est une vue de côté du pivot fémoral de l'invention, la figure 5b une vue en coupe selon le plan B-B de la figure 5a, la figure 5c une vue de dessus et la figure 5d une vue de dessous du pivot fémoral de la figure 5a ;
- les figures 6a, 6b et 6c représentent différentes vues en perspective du pivot fémoral de l'invention et les figures 6c et 6e illustrent des vues à échelle agrandie des détails g et k des figures 6b et 6e ;
- la figure 7a représente les contours en projection dans un plan latéral d'un jeu de dix pivots fémoraux de tailles différentes et la figure 7b représente les contours de ce jeu en projection dans un plan frontal ;
- la figure 7c est une vue en coupe selon le plan C-C, la figure 7d une vue en coupe selon le plan D-D et la figure 7e une vue en coupe selon le plan E-E d'un même pivot fémoral T5 de la figure 7a.

### Liste des repères :

| | |
|---|---|
| 1 pivot fémoral | 2 cavité col |
| 3 partie métaphysaire | 4 partie diaphysaire |
| 5 face antérieure | 6 face postérieure |
| 7 face interne | 8 face externe |
| 9 partie de raccordement | 10 surface frontale supérieure |
| 11 surface réception col | 12 tronc de cône générateur |
| 13 extrémité inférieure | 14 face externe métaphysaire |
| 15 face externe diaphysaire | 16 canal longitudinal |
| 17 face antérieure diaphysaire | 18 extrémité supérieure |
| 19 rainures verticales | 20 face antérieure métaphysaire |
| 21 nervures horizontales | 22 face interne métaphysaire |
| 23 face interne diaphysaire | 24 canal longitudinal |
| 25 rainures horizontales | 26 orifice taraudé |
| 27 face postérieure diaphysaire | 28 rainures verticales |
| 29 face postérieure métaphysaire | 30 nervures horizontales |
| 31 extrémité supérieure | 32 base |
| 33 sommet | 34 grande ellipse |
| 35 petite ellipse | |

### Description de l'invention

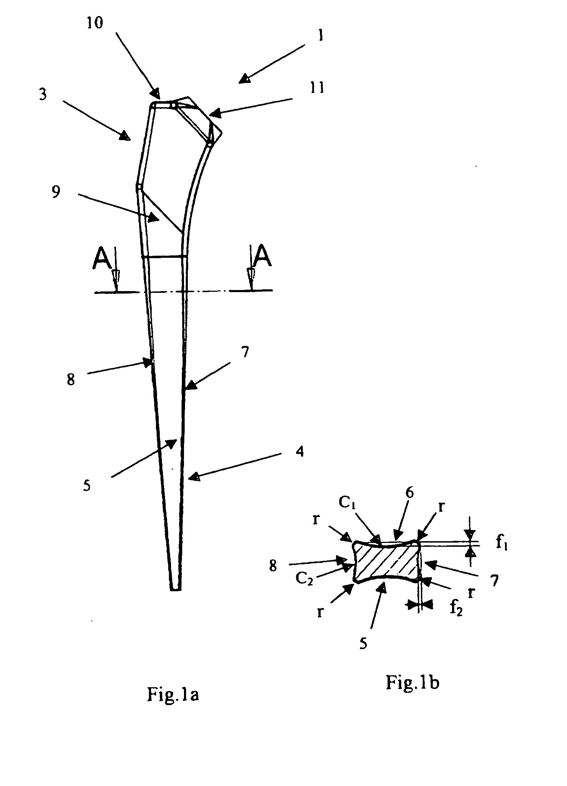

Les figures 1 à 2 illustrent un pivot fémoral selon un premier mode de réalisation de l'invention, plus particulièrement du type à être implanté avec ciment. Le pivot fémoral 1 est en effet une tige fémorale exempte de col, il se compose, en partie haute, d'une partie métaphysaire 3 ayant une longueur égale à environ 1/3 de la longueur du pivot prolongée vers le bas par une partie diaphysaire 4 (de longueur égale à environ 2/3 de la longueur du pivot). La surface de la section transversale du pivot fémoral va en croissant vers le haut, la partie diaphysaire 4 et métaphysaire 3 se raccordant sans discontinuité.

Dans ce qui suit, les faces postérieure et antérieures sont définies pour un pivot fémoral de prothèse de hanche droite (tel que visible sur les figures). Il sera bien compris par la suite qu'une face antérieure de pivot fémoral droit deviendra une face postérieure pour un pivot fémoral gauche et qu'une face postérieure de pivot fémoral droit deviendra une face postérieure dans le cas d'un pivot fémoral pour une prothèse de hanche gauche.

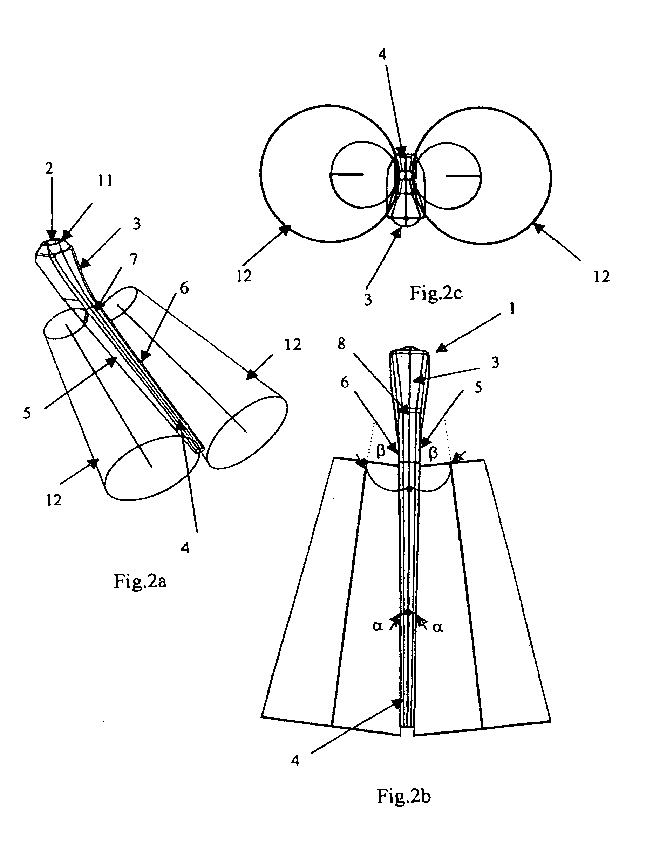

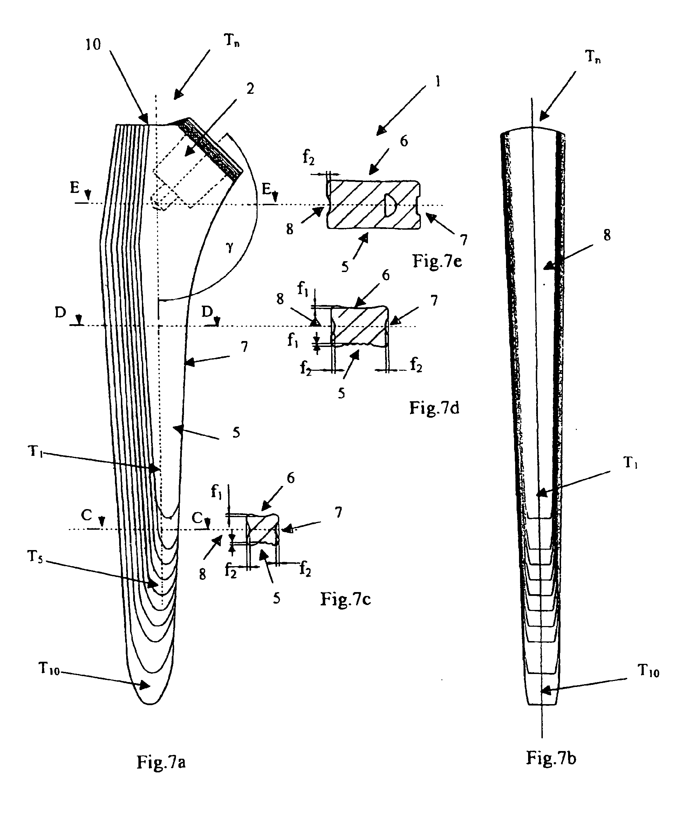

Le pivot fémoral 1 présente un profil longitudinal effilé, les faces antérieure 5 et postérieure 6 ont une même forme, elles sont évasées à partir de l'extrémité inférieure de la partie diaphysaire 4 en direction de la partie métaphysaire 3 (fig. 1a). Telle que vue de face, la partie diaphysaire de la face antérieure présente une forme générale de triangle isocèle à sommet arrondi, son axe de symétrie s'étendant selon l'axe longitudinal vertical de la partie diaphysaire. La partie diaphysaire 4 est liée à la partie métaphysaire 3 par une partie de raccordement 9. La partie métaphysaire s'étend également en s'évasant vers le haut, mais selon une direction inclinée par rapport à l'axe longitudinal du pivot, direction qui suit généralement celle de l'axe du col. Le col fémoral (non représenté sur les dessins) est avantageusement fixé de manière amovible dans une cavité de col 2 (fig.2a) pratiquée sur une certaine profondeur à partir de la surface de réception de col 11. A titre d'exemple, l'angle y fait par l'axe du col et l'axe longitudinal vertical de la partie diaphysaire est d'environ 135° (fig.7a).

La face interne 7 du pivot fémoral 1 est mieux visible à la figure 2a et sa face externe 8 à la figure 2b où l'on remarque le profil longitudinal effilé, les faces antérieure 5 et postérieure 6 présentant une faible inclinaison avec le plan de symétrie du pivot fémoral 1 sur la partie diaphysaire 4, elles s'évasant encore plus au niveau de la partie métaphysaire 3.

Le pivot fémoral 1 présente une section transversale (section réalisée avec un plan de coupe perpendiculaire à l'axe longitudinal du pivot) de forme générale rectangulaire, les deux côtés les plus longs correspondant aux faces antérieure 5 et postérieure 6 et les côtés courts aux faces interne 7 et externe 8 (fig. 1a et 1 b).

Selon l'invention et tel que mieux visible à la figure 1b, les côtés de la section transversale de la partie diaphysaire 4 sont recourbés vers l'intérieur de la section, en suivant des arcs de courbes planes, notamment un arc d'ellipse C1 pour les côtés correspondant aux faces antérieure 5 et postérieure 6 et en suivant un arc d'ellipse C2 pour les côtés des faces interne 7 et externe 8.

Dans une mode préférée de réalisation de l'invention et tel que mieux visible aux figures 2a à 2c, la courbure des faces antérieure 5 et postérieure 6 est réalisée en intersectant la surface initialement plane de la face antérieure 5 et celle de la face postérieure 6 avec un tronc de cône d'axe incliné par rapport à l'axe longitudinal du pivot fémoral. La face antérieure 5 et la face postérieure 6 font chacune un angle α avec l'axe longitudinal du pivot, l'axe du tronc de cône générateur 12 fait un angle β avec le plan de symétrie vertical du pivot fémoral 1 et sa génératrice est tangente au plan de la face antérieure 5, respectivement de la face postérieure 6 sur toute sa longueur. A titre d'exemple l'angle α est compris entre 5° et 15°, et est de préférence égal à 7, 3° et l'angle β est compris entre 5° et 15°, et est de préférence égal à 7,1°. Le rayon de la grande base du tronc de cône générateur 12 est compris entre 35mm et 45mm, de préférence égal à 40,4mm et le rayon de la petite base du tronc de cône générateur 12 est compris entre 14mm et 24mm, de préférence égal à 19,6mm.

Tel que mieux visible à la figure 1b, les quatre coins de la section transversale de la partie diaphysaire 4 du pivot fémoral sont arrondis, la valeur du rayon de raccordement étant dans l'exemple représenté de 3,5 mm.

La courbure de l'une des faces antérieure 5 et postérieure 6 présente une flèche f1 comprise entre 0,5 mm et 4 mm et de préférence égale à 1,8 mm.

La courbure des faces interne 7 et externe 8 est obtenue en pratiquant un canal conique incliné selon l'inclinaison de la face interne, respectivement celle de la face externe, le rayon à la base du cône étant compris entre 12m et 32mm, de préférence 22mm. A titre d'exemple, la flèche f2 de leur arc C2 est comprise entre 0,5 mm et 4 mm et de préférence égale à 1,8 mm.

Dans une variante de réalisation de l'invention, la courbure des faces interne 7 et externe 8 peut être obtenue avec un tronc de cône générateur incliné, la petite base étant positionnée proche de l'extrémité 13 de la partie diaphysaire 4. La courbure des faces internes et externes commence en la partie supérieure de la partie daphysaire 4, au niveau de sa jonction avec la partie métaphysaire 3 et s'arrête à environ 10 à 20 mm au dessus de l'extrémité distale de la partie diaphysaire 4.

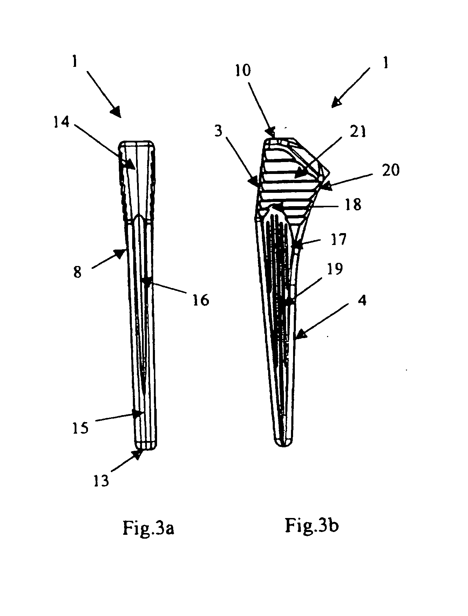

Les figures 3 à 7 illustrent un pivot fémoral selon un deuxième mode de réalisation de l'invention, plus particulièrement du type à être implanté sans utiliser de ciment. Les faces antérieure 5, postérieure 6 ainsi que interne 7 et externe 8 ont les mêmes formes et sont générées de la même manière que dans l'exemple décrit en référence aux figures précédentes, ainsi que la partie diaphysaire 4 et métaphysaire 3 et leurs éléments précédemment décrits et de ce fait, ces parties et éléments ont gardé les mêmes numéros de référence.

Tel que représenté à la figure 3a, la face externe 8 du pivot fémoral 1 comprend une face externe métaphysaire 14 ayant une surface lisse prolongée vers la bas par une partie externe diaphysaire 15 munie, elle, d'un canal longitudinal 16 vertical conique s'étendant sur la majeure partie de la longueur de celle-ci. Le canal longitudinal 16 présente une section transversale ayant une forme générale en U et une profondeur décroissante vers l'extrémité inférieure 13 de la partie diaphysaire 4. La largeur du canal longitudinal 16 va en diminuant vers la bas, et se termine par une forme pointue, à fond arrondi, fermée à quelques cm au dessus de l'extrémité inférieure 13. Cette forme a été judicieusement choisie afin de faciliter l'insertion de l'implant dans la cavité médullaire du fémur, mais également le retrait de l'implant en cas de besoin.

La figure 3b illustre une vue de côté du pivot fémoral de la figure 3a, où l'on remarque la face antérieure diaphysaire 17 qui est munie de cinq rainures verticales 19 parallèles à l'axe longitudinal du pivot fémoral 1. Les cinq rainures verticales 19 sont situées à égale distance l'une de l'autre et l'on note : une rainure centrale longue s'étendant sur toute la longueur de la face antérieure diaphysaire 17, deux rainures intermédiaires plus courtes adjacentes à la première, situées de part et d'autre de celle-ci ainsi que deux rainures d'extrémité adjacentes en étant situées à l'extérieur des rainures intermédiaires et étant encore plus courtes que les précédentes. Ces rainures verticales 19 sont non débouchantes au niveau de leurs extrémités qui se raccordent à la surface de la partie antérieure diaphysaire par des zones arrondies. La face antérieure diaphysaire se termine par une extrémité supérieure 18 arrondie selon un grand rayon de raccordement.

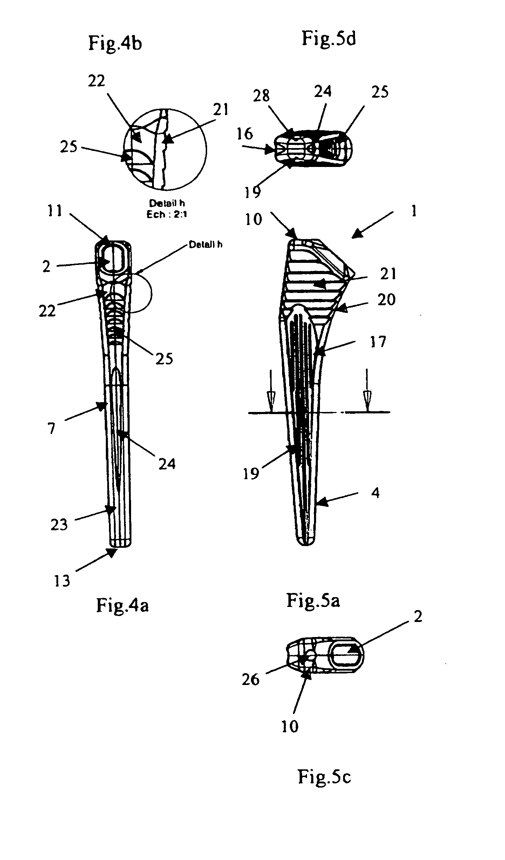

La face antérieure métaphysaire 20 est munie de plusieurs nervures horizontales 21 parallèles entre elles et perpendiculaires à l'axe longitudinal du pivot fémoral. Dans l'exemple représenté aux figures, huit nervures horizontales 21 sont uniformément réparties sur toute la surface de la face antérieure métaphysaire 20, les deux dernières étant interrompues par la zone arrondie en creux de l'extrémité supérieure 18 de la face antérieure diaphysaire 17. Les rainures horizontales 21 se trouvent l'une dans le prolongement de l'autre présentant un profil en dents de scie, tel que mieux visible à la figure 4b.

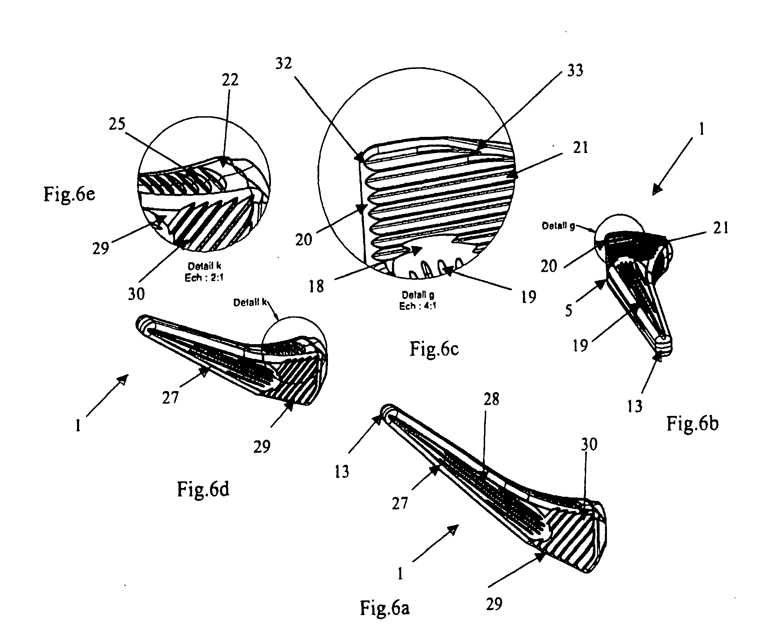

La figure 4a illustre une vue frontale de la face interne 7 du pivot fémoral 1 où l'on remarque une face interne métaphysaire 22 ayant une surface nervurée prolongée vers la bas par une partie interne diaphysaire 23 munie d'un canal longitudinal 24 vertical conique s'étendant sur la majeure partie de la longueur de celle-ci. Le canal longitudinal 24 présente une section transversale ayant une forme générale en U et une profondeur décroissante vers l'extrémité inférieure 13 de la partie diaphysaire 4. La largeur du canal longitudinal 24 va en diminuant vers la bas, et se termine par une forme pointue, à fond arrondi, fermée à quelques cm au dessus de l'extrémité inférieure 13. Cette forme a été judicieusement choisie afin de faciliter l'insertion de l'implant dans la cavité médullaire du fémur, mais également le retrait de l'implant en cas de besoin. La face interne métaphysaire comporte plusieurs rainures horizontales 25 en creux, cinq dans l'exemple représenté, qui sont en forme d'écailles de poisson (fig. 6e) et sont uniformément réparties sur la surface de la face interne métaphysaire 22.

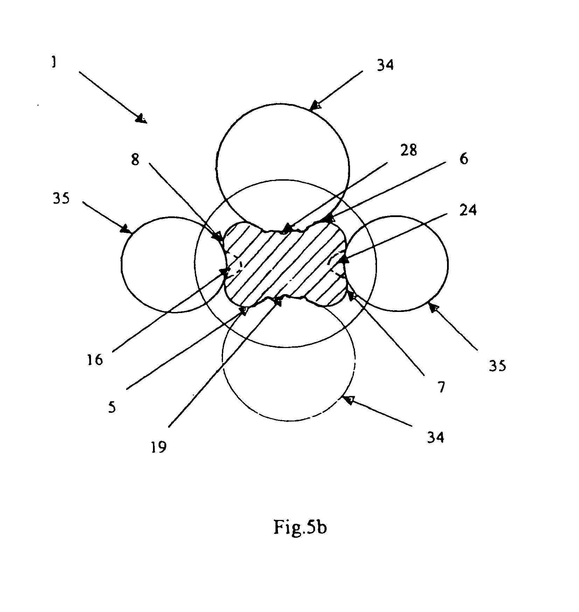

La figure 5b illustre la section transversale obtenue avec le plan B-B de la figure 5a où l'on remarque la forme rectangulaire à contour rogné et côtés arrondis, les canaux longitudinaux 16, 24, ainsi que les rainures verticales 19 et 28 étant également visibles. Il a été représenté sur la figure 5b deux grandes ellipses 34, tangentes aux faces antérieure 5 et postérieure 6, et deux petites ellipses 35 tangentes aux faces interne 7 et externe 8, en étant obtenues avec un plan de coupe perpendiculaire à l'axe longitudinal du pivot fémoral 1. Les grandes ellipses 34 sont obtenues en intersectant les plans des faces antérieure 5 et postérieure 6 avec des troncs de cône 12 identiques et les petites ellipses 35 sont obtenues en intersectant les plans des faces interne 7 et externe 8 avec des cônes inversés identiques.

La figure 5c illustre une vue de dessus du pivot fémoral de la figure 5a, où l'on remarque la cavité 2 destinée à recevoir un col amovible, ainsi qu'un orifice taraudé 26 pratiqué à partir de la surface frontale supérieure 10. L'orifice taraudé 26 est prévu pour coopérer avec un organe extracteur en cas de reprise de prothèse, mais également pour positionner un organe impacteur fémoral en cas de mise en place de la prothèse.

La figure 5d montre une vue de dessous du pivot fémoral de la figure 5c où l'on remarque les canaux longitudinaux 16,24, les rainures longitudinales 19,28 et les rainures horizontales 25.

Les figures 6a, 6d et 6e illustrent mieux la face postérieure du pivot fémoral selon le deuxième mode de réalisation de l'invention où l'on remarque la face postérieure diaphysaire 27 qui est munie de cinq rainures verticales 28 parallèles à l'axe longitudinal du pivot fémoral 1. Les cinq rainures verticales 28 sont situées à égale distance l'une de l'autre et l'on note : une rainure centrale longue s'étendant sur toute la longueur de la face postérieure diaphysaire 27, deux rainures intermédiaires plus courtes adjacentes à la première, situées de part et d'autre de celle-ci ainsi que deux rainures d'extrémité adjacentes en étant situées à l'extérieur des rainures intermédiaires et étant encore plus courtes que les précédentes. Ces rainures verticales 28 sont non débouchantes au niveau de leurs extrémités qui se raccordent à la surface de la partie antérieure diaphysaire par des zones arrondies. La face postérieure diaphysaire 27 se termine par une extrémité supérieure 31 arrondie selon un grand rayon de raccordement.

La face postérieure métaphysaire 29 du pivot fémoral de prothèse de hanche droite (ou face antérieure métaphysaire dans le cas d'un pivot fémoral pour une prothèse de hanche gauche) est munie de plusieurs nervures horizontales 30 parallèles entre elles et perpendiculaires à l'axe longitudinal du pivot fémoral. Dans l'exemple représenté aux figures, huit nervures horizontales 30 sont uniformément réparties sur toute la surface de la face postérieure métaphysaire 29, les deux dernières étant interrompues par la zone arrondie en creux de l'extrémité supérieure 31 de la face postérieure diaphysaire 27 (ou face antérieure diaphysaire dans le cas d'un pivot fémoral pour une prothèse de hanche gauche). Les rainures horizontales 30 se trouvent l'une dans le prolongement de l'autre présentant un profil triangulaire en dents de scie, tel que mieux visible à la figure 6e.

La face antérieure 5 est représentée par la vue en perspective de la figure 6b et le détail des nervures horizontales 21 de la partie antérieure métaphysaire à la figure 6c. Les nervures horizontales 21 sont agencées l'une dans la continuité de l'autre sur toute la surface de la partie antérieure métaphysaire 20. Elles comportent une base 32 arrondie avoisinant la face externe et s'étendent horizontalement vers la face interne où elles se terminent par un sommet 33 pointu.

Dans l'exemple représenté le pivot fémoral comporte cinq rainures verticales 19, 28, le nombre de rainures dépendant toutefois de la taille du pivot fémoral, ce nombre étant compris entre trois et sept rainures verticales. De la même manière le nombre des nervures et rainures horizontales 21, 25, 30 varie en fonction de la taille du pivot fémoral, ce nombre étant compris entre 5 et 10 nervures ou rainures horizontales pour une gamme de douze pivots.

Aux figures 7a et 7b on a représenté les projections dans un plan latéral, respectivement frontal, d'une gamme Tn de dix pivots fémoraux de tailles différentes référencés T1 à T10. Ainsi le contour du pivot fémoral T10 correspond au contour extérieur tracé et le contour du pivot fémoral T1 correspond au contour intérieur tracé, les tailles intermédiaires étant représentées par les contours intermédiaires. Tel que visible à la figure 7a, les contours croissent en proportion homothétique pour toutes les dimensions de son profile, hormis pour a face interne 7 dont le profil reste constant pour toutes les tailles. La longueur des pivots augmente également de manière homothétique selon la taille du pivot.

Les figures 7c, 7d et 7e illustrent des sections transversales d'un pivot T5 (de taille 5) où l'on remarque les flèches f1 et f2 qui ont une valeur constante pour toutes les sections d'un même pivot fémoral, en l'occurrence T5. A titre d'exemple, la valeur de la flèche f1 est de 0,75 mm et celle de la flèche f2 de 1 mm.

La valeur des flèches f1 et f2 est évolutive avec la taille du pivot. Elle est en moyenne de 1,8 mm pour chacune des flèches f1 et f2 et elle peut aller de 0,5 mm à 4 mm en fonction du nombre de pivots d'une gamme de pivots fémoraux selon l'invention.

Le pivot fémoral de l'invention est avantageusement réalisé, lorsqu'il est destiné à être implanté avec ciment, en un alliage d'acier inoxydable, par exemple M30 et, lorsqu'il est destiné à être implanté sans ciment, en un alliage de titane, par exemple du TA6V. Le pivot fémoral de l'invention peut avantageusement être réalisé par une technique de moulage et/ou de forgeage, dans quel cas, une opération de fraisage supplémentaire peut être envisagée pour réaliser les diverses nervures, rainures et canaux longitudinaux.

Les rainures verticales 19, 28 les canaux longitudinaux 16, 24 ainsi que les nervures horizontales 21, 30 et rainures horizontales 25 augmentent la surface de contact de l'implant avec l'os et assurent une meilleure résistance aux sollicitations mécaniques de l'implant.

De surcroît, les nervures horizontales 21, 30 et rainures horizontales 25 subissent un traitement de surface et/ou sont recouvertes d'une couche de revêtement en hydroxyapatite de calcium (HAC) et/ou titane poreux afin de permettre à l'os de coloniser le pivot fémoral et favoriser ainsi la tenue dans le temps de l'implant. Ce revêtement pourra être ajouté sur toute ou partie de la surface du pivot fémoral.

D'autres variantes et modes de réalisation de l'invention peuvent être envisagés sans sortir du cadre de l'invention telle que délimitée dans les revendications.

## Revendications

1. Pivot fémoral (1) pour une tige fémorale de prothèse de hanche, ledit pivot comportant une partie métaphysaire (3) proximale prolongée vers la bas par une partie diaphysaire (4) distale, chacune des parties métaphysaire (3) et diaphysaire (4) présentant une section transversale d'ancrage dans le fémur ayant un contour extérieur de forme générale rectangulaire, section qui va en se rétrécissant de la partie proximale vers la partie distale et ledit pivot étant **caractérisé en ce que** la section transversale d'ancrage présente un contour rogné et que les côtés du contour sont recourbés vers son centre suivant un arc d'ellipse sur la majeure partie de la partie diaphysaire (4).

2. Pivot fémoral (1) selon la revendication 1, **caractérisé en ce que** la surface de la face antérieure et celle de la face postérieure de la partie diaphysaire (4) sont obtenues chacune par l'intersection d'un plan incliné faisant un angle α avec le plan de symétrie du pivot avec un tronc de cône générateur (12) d'axe incliné faisant un angle p avec le plan de symétrie du pivot, la génératrice du cône étant tangente au plan de la face antérieure (5), respectivement de la face postérieure (6) sur toute sa longueur.

3. Pivot fémoral (1) selon l'une des revendications précédentes, **caractérisé en ce que**, pour une section transversale prédéfinie, les côtés des faces antérieure (5) et postérieure (6) présentent un même rayon de courbure qui est supérieur à celui des faces interne (7) et externe (8).

4. Pivot fémoral (1) selon l'une des revendications précédentes, **caractérisée en ce que** le contour extérieur de la section transversale d'ancrage présente des coins arrondis.

5. Pivot fémoral (1) selon l'une des revendications précédentes, **caractérisée en ce que** la partie métaphysaire (3) présente une forme évasée en direction d'une cavité de col (2).

6. Pivot fémoral (1) selon l'une des revendications précédentes, **caractérisée en ce que** la partie métaphysaire (3) présente des nervures horizontales (21,30) et des rainures horizontales (25) sensiblement sur la totalité de la surface de ses faces interne (7), antérieure (5) et postérieure (6).

7. Pivot fémoral (1) selon l'une des revendications précédentes, **caractérisée en ce qu'**il comporte une cavité (2) de réception d'un col amovible.

8. Pivot fémoral (1) selon l'une des revendications précédentes, **caractérisé en ce que** la partie métaphysaire comprend un orifice taraudé de réception d'organe extracteur ou impacteur.

9. Gamme de pivots fémoraux (Tn), **caractérisé en ce qu'**elle comprend une pluralité de pivots fémoraux selon l'une des revendications précédentes, les dimensions au moins de leur longueur et des paramètres définissant le profil de la section transversale pour les faces antérieure (5), postérieure (6) et externe (8) de chaque pivot fémoral (1) sont établies en progression homothétique.

10. Tige fémorale comportant un pivot fémoral (1) selon l'une des revendications 1 à 8.

11. Prothèse de hanche comportant une tige fémorale selon la revendication 10.

## Claims

1. Femoral pivot (1) for a hip prosthesis femoral shaft, said pivot comprising a proximale metaphyseal portion (3) extended at the bottom by a distale diaphyseal portion (4), each of the metaphyseal (3) and diaphyseal (4) portions haring an anchoring cross-section in the femur having an outer contour with a general rectangular shape, said cross-section refracting from the proximal portion to the distal portion and said pivot being **characterised in that** the anchoring cross-section has a trimmed contour and that the sides of the contour are curved towards the centre thereof along an elliptical arc on most of the diaphyseal portion (4).

2. Femoral pivot (1) according to claim 1, **characterised in that** the surface of the front face and that of the rear face of the diaphyseal portion (4) are each obtained by intersecting an inclined plane forming an angle α with the plane of symmetry of the pivot with a generating line truncated cone (12) having an inclined axis forming an angle β with the plane of symmetry of the pivot, the generating line of the cone being tangent with the plane of the front face (5), or the rear face (6), over the entire length thereof.

3. Femoral pivot (1) according to any of the above claims, **characterised in that**, for a predefined cross-section, the sides of the front (5) and rear (6) faces have the same radius of curvature which is greater than that of the inner (7) and outer (8) faces.

4. Femoral pivot (1) according to any of the above claims, **characterised in that** the outer contour of the anchoring cross-section has rounded corners.

5. Femoral pivot (1) according to any of the above claims, **characterised in that** the metaphyseal portion (3) have a flared shape towards a neck cavity (2).

6. Femoral pivot (1) according to any of the above claims, **characterized in that** the metaphyseal portion (3) has horizontal ribs (21, 30) and horizontal grooves (25) substantially on the entire surface of the inner (7), front (5) and rear (6) faces thereof.

7. Femoral pivot (1) according to any of the above claims, **characterised in that** it comprises a cavity (2) for receiving a removably neck.

8. Femoral pivot (1) according to any of the above claims, **characterised in that** the metaphyseal portion comprises a threaded orifice for receiving an extracting or imparting member.

9. Range of femoral pivots (Tn), characterises in that it comprised a plurality of femoral pivots according to any of the above claims, the dimensions at least of the length thereof and parameters defining the profile of the cross-section of the front (5), rear (6) and outer (8) faces of each femoral pivot (1) are determined in homothetic progression.

10. Femoral shaft comprising a femoral pivot (1) according to any of claims 1 to 8.

11. Hip prostheses comprise a femoral shaft according to claim 10.

## Patentansprüche

1. Femurzapfen (1) für einem Femurstift einer Hüftprothese, wobei der Zapfen ein proximales metaphysären Teil (3) umfasst, das durch ein distales diaphysäres Teil (4) nach unten hin verlängert ist, wobei das metaphysäre Teil (3) und das diaphysäre Teil (4) einen Verankerungsquerschnitt mit einem generell rechteckigen Außenumriss im Femur aufweisen, wobei sich der Querschnitt vom proximalen Teil zum distalen Teil hin verengt und wobei der Zapfen **dadurch gekennzeichnet ist, dass** der Verankerungsquerschnitt einen gestutzten Umriss aufweist und dass die Seiten des Umrissen zu dessen Mitte hin nach einem Ellipsenbogen am Hauptteil des diaphysären Teils (4) gebogen sind.

2. Femurzapfen (1) nach Anspruch 1, **dadurch gekennzeichnet, dass** die Oberfläche der Vorderseite und die Oberfläche der Hinterseite des diaphysären Teils (4) jeweils erhalten werden durch die Schnittfläche einer schräge Ebene, die einen Winkel ß mit der Symmetrieebene des Zapfens bildet, mit einem den Schrägwinkel erzeugenden Kegelstumpf (12), der einen Winkel ß mit der Symmetrieebene des Zapfens bildet, wobei der Erzeuger des Kegels über seine gesamte Länge tangential zur Ebene der Vorderseite (5) und der Hinterseite (6) ist.

3. Femurzapfen (1) nach einen der vorhergehenden Ansprüche, **dadurch gekennzeichnet, dass**, für einen vordefinierten Querschnitt, die Seiten der Vorderseite (5) und der Hinterseite (6) denselben Krümmungsradius aufweisen, der größer als derjenige von der internen Seite (7) und von der externen Seite (8) ist.

4. Femurzapfen (1) nach einem der vorhergehenden Ansprüche, **dadurch gekennzeichnet, dass** der Außenumriss des Verankerungsquerschnitts abgerundete Ecken aufweiset.

5. Femurzapfen (1) nach einem der vorhergehenden Ansprüche, **dadurch gekennzeichnet, dass** das metaphysäre Teil (3) eine sich erweiternde Form in Richtung eines Halshohlraums (2) aufweist.

6. Femurzapfen (1) nach einem der vorhergehenden Ansprüche, **dadurch gekennzeichnet, dass** das metaphysäre Teil (3) im Wesentlichen auf der gesamten Oberfläche seiner internen Seite (7), seiner Vorderseite (5) und seiner Hinterseite (6) horizontale Rippe (21, 30) und horizontale Nuten (25) aufweist.

7. Femurzapfen (1) nach einem der vorhergehenden Ansprüche, **dadurch gekennzeichnet, dass** er einen Hohlraum (2) für die Aufnahme eines entfernbaren Halses umfasst.

8. Femurzapfen (1) nach einem der vorhergehenden Ansprüche, **dadurch gekennzeichnet, dass** das metaphysäre Teil eine Öffnung mit Gewinde zur Aufnahme eines Extraktions- oder Impaktionsorgans umfasst.

9. Sortiment an Femurzapfen (Tn), **dadurch gekennzeichnet, dass** es mehrere Femurzapfen nach einem der vorhergehenden Ansprüche umfasst, wobei die Abmessungen zumindest ihrer Länge und der Parameter, die das Profil des Querschnitts für die Vorderseite (5), die Hinterseite (6) und die externe Seite (8) von jedem Femurzapfen (1) definieren, im homothetischen Fortschritt erstellt sind.

10. Femurstift umfassend einen Femurzapfen (1) nach einem der Ansprüche 1 bis 8.

11. Hüftprothese umfassen einen Femurstift nach Anspruch 10.
